(19) Europäisches Patentamt / European Patent Office / Office européen des brevets

(11) **EP 4 198 014 A1**

(12) **EUROPÄISCHE PATENTANMELDUNG**

(43) Veröffentlichungstag:
**21.06.2023   Patentblatt 2023/25**

(21) Anmeldenummer: **21215858.8**

(22) Anmeldetag: **20.12.2021**

(51) Internationale Patentklassifikation (IPC):
**C07C 51/09** (2006.01)   **C07C 67/38** (2006.01)
**C07C 67/56** (2006.01)   **C07C 53/126** (2006.01)
**C07C 69/24** (2006.01)   **B01D 61/00** (2006.01)

(52) Gemeinsame Patentklassifikation (CPC):
(C-Sets verfügbar)
**B01D 61/027; B01D 71/52; C07C 51/09;**
**C07C 67/38; C07C 67/56;** B01D 2311/04;
B01D 2311/06; B01D 2311/263        (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Evonik Operations GmbH**
**45128 Essen (DE)**

(72) Erfinder:
• **KUCMIERCZYK, Peter**
**44628 Herne (DE)**
• **FRIDAG, Dirk**
**45721 Haltern am See (DE)**
• **SCHÄPERTÖNS, Marc**
**45657 Recklinghausen (DE)**
• **GLUTH, Frederik**
**45472 Mülheim an der Ruhr (DE)**

• **DREIMANN, Jens Martin**
**44141 Dortmund (DE)**
• **FRANKE, Robert**
**45772 Marl (DE)**
• **KNOSSALA, Johannes**
**46514 Gahlen (DE)**
• **BRÄCHER, Alexander**
**45721 Haltern am See (DE)**
• **SALE, Anna Chiara**
**45657 Recklinghausen (DE)**
• **MARKOVIC, Ana**
**45721 Haltern am See (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54) **VERFAHREN ZUR HERSTELLUNG VON CARBONSÄUREN ODER DEREN SALZEN UND ABTRENNUNG DES HOMOGENEN KATALYSATORSYSTEMS UNTER VERWENDUNG EINER MEMBRAN UMFASSEND EINE TRENNAKTIVE SCHICHT AUS PAEK UND EINER UNTERSTRUKTUR, DIE PAEK UMFASST**

(57)    Gegenstand der Erfindung ist ein Verfahren zur Herstellung von Carbonsäuren oder deren Salzen durch Hydrolyse oder Verseifung eines Esters, der durch Alkoxycarbonylierung eines C2- bis C20-Kohlenwasserstoffs mit mindestens einer Mehrfachbindung, vorzugsweise mit mindestens einer olefinischen Doppelbindung, erhalten wird, bei dem das eingesetzte homogene Katalysatorsystem mittels Membrantrennung unter Verwendung einer Membran umfassend eine trennaktive Schicht aus PAEK und einer Unterstruktur, die PAEK umfasst, aus dem Produktgemisch abgetrennt wird. In einer Weiterbildung der vorliegenden Erfindung wird der so gebildete Ester durch Umesterung zu einem anderen Ester umgesetzt und dann hydrolysiert oder verseift.

(52) Gemeinsame Patentklassifikation (CPC): (Forts.)

C-Sets
**C07C 51/09, C07C 53/126;**
**C07C 67/38, C07C 69/24;**
**C07C 67/56, C07C 69/24**

**Beschreibung**

Die vorliegende Erfindung betrifft ein Verfahren zur Herstellung von Carbonsäuren oder deren Salzen durch Hydrolyse oder Verseifung eines Esters, der durch Alkoxycarbonylierung eines C2- bis C20-Kohlenwasserstoffs mit mindestens einer Mehrfachbindung, vorzugsweise mit mindestens einer olefinischen Doppelbindung, erhalten wird, bei dem das eingesetzte homogene Katalysatorsystem mittels Membrantrennung unter Verwendung einer Membran umfassend eine trennaktive Schicht aus PAEK und einer Unterstruktur, die PAEK umfasst, aus dem Produktgemisch abgetrennt wird. In einer Weiterbildung der vorliegenden Erfindung wird der so gebildete Ester durch Umesterung zu einem anderen Ester umgesetzt und dann hydrolysiert oder verseift.

Die Herstellung von Carbonsäuren in der großindustriellen Chemie erfolgt zum größten Teil über die Hydroformylierung zur Herstellung eines Aldehyds mit nachfolgender Oxidation des Aldehyds zur Carbonsäure. Wenngleich die Herstellung von Carbonsäuren im Wege der Hydroformylierung mit nachfolgender Oxidation ein seit Jahrzehnten industriell etablierter und bewährter Prozess ist, weist er immer noch Verbesserungspotential auf (vgl. DE 100 10 771 C1 oder EP 1 854 778 A1). Ein Problem bei dieser Syntheseroute ist, dass bei der Hydroformylierung Übergangsmetall-haltige Katalysatorsysteme eingesetzt werden, die üblicherweise teuer sind oder kostenintensiv hergestellt werden müssen. Ein weiteres Problem ist die Bildung von Nebenprodukten, die sich selbst dann zu beobachten ist, wenn kein Katalysator eingesetzt wird und/oder die Reaktionsbedingungen vergleichsweise mild sind. Ein weiteres Problem ist der erhöhte Bedarf an Sicherheitsausrüstung bei der Oxidation von Aldehyden, da in diesem Schritt Sauerstoff in reiner Form oder durch Luft der organischen Reaktionsmischung zugeführt wird. Die dadurch erzeugte explosive Atmosphäre muss gesondert und erschwert überwacht und geregelt werden.

Zur Vermeidung von Nebenreaktionen und zur Erhöhung der Selektivität ist in der Literatur vorgeschlagen worden Alkali- und/oder Erdalkalimetallverbindungen zur Reaktionslösung zu geben. Nachteil dieser Variante ist aber, dass diese Verbindungen eine inhibierende Wirkung auf die Oxidation der Aldehyde haben und deshalb längere Reaktionszeiten notwendig sind, um ausreichende Umsätze zu erzielen.

Der vorliegenden Erfindung lag daher die Aufgabe zu Grunde, einen alternativen Syntheseweg für die Herstellung von Carbonsäuren oder deren Salzen anzugeben, mit der sich die gewünschten Carbonsäuren bzw. deren Salze relativ einfach erschließen lassen und bei der auf die Anwesenheit von Alkali- und/oder Erdalkalimetallverbindungen verzichtet werden kann. Eine weitere wichtige Zielsetzung ist, dass das Herstellungsverfahren im großindustriellen Maßstab durchführbar ist. Es gilt die klassische Synthese durch eine andere Synthesetechnologie zu ersetzen, welche die Produkte des bekannten Verfahrens in besserer Qualität liefert. Darüber hinaus soll der neu zu schaffende Syntheseweg weniger unerwünschte Nebenprodukte bilden. Eine weitere Aufgabe der vorliegenden Erfindung war die Bereitstellung eines neuartigen Membranmaterials für die Abtrennung des homogen gelösten Katalysatorsystems.

Gelöst wird diese Aufgabe durch ein zweistufiges Verfahren in welchem in einem ersten Schritt in einer Alkoxycarbonylierung ein Kohlenwasserstoff mit mindestens einer Mehrfachbindung mit Kohlenmonoxid und einem Alkohol zu einem Ester umgesetzt wird und in welchem in einem zweiten Schritt in einer Hydrolyse oder Verseifung der Ester in Anwesenheit eines sauren Katalysators bzw. eines Verseifungsmittels zu der gewünschten Carbonsäure bzw. zu dem gewünschten Carbonsäuresalz umgesetzt wird. Dabei wird in dem zweiten Schritt der ursprünglich eingesetzte Alkohol teilweise wieder freigesetzt.

Erfindungsgemäß umfasst das Verfahren zur Herstellung einer Carbonsäure oder eines Carbonsäuresalzes die folgenden Schritte:

a) Herstellung eines Esters durch Umsetzen (Carbonylieren) eines C2- bis C20-Kohlenwasserstoffs mit mindestens einer Mehrfachbindung, vorzugsweise mindestens einer olefinischen Doppelbindung mit Kohlenmonoxid und mit einem Alkohol A in Gegenwart eines homogenen Katalysatorsystems in einer Reaktionszone unter Erhalt eines Produktgemisches;

b) Durchführen einer Membrantrennung zur Abtrennung des homogenen Katalysatorsystems aus dem Produktgemisch, wodurch das homogene Katalysatorsystem und nicht umgesetzter Kohlenwasserstoff und/oder nicht umgesetzter Alkohol A, vorzugsweise nicht umgesetzter Alkohol A im Retentat angereichert werden und der in Schritt a) gebildete Ester im Permeat angereichert wird, wobei bei der Membrantrennung eine Membran eingesetzt wird, die eine auf einer Unterstruktur aufgebrachte trennaktive Schicht umfasst, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht;

c) Abtrennen des in Schritt a) gebildeten Esters aus dem Permeat in mindestens einem Trennverfahrensschritt, ausgewählt aus thermischer Trennung, beispielsweise Destillation, Extraktion, Kristallisation und Membrantrennung;

d) Hydrolyse oder Verseifen des in Schritt a) hergestellten Esters in Anwesenheit eines sauren Katalysators oder eines Verseifungsmittels unter Erhalt eines Reaktionsgemisches, welches zumindest die Carbonsäure oder deren Salz, den abgespaltenen Alkohol A, Wasser und nicht umgesetzte Ester umfasst;

e) Abtrennen der in Schritt d) gebildeten Carbonsäure oder deren Salz in mindestens einem Trennverfahrensschritt, ausgewählt aus thermischer Trennung, beispielsweise Destillation, Extraktion, Kristallisation und Membrantrennung.

Thermische Trennung im Sinne der vorliegenden Erfindung meint ein Trennverfahren, bei dem die Trennung anhand des Siedepunktes erfolgt.

Die bei der Umsetzung in Schritt a) eingesetzten Kohlenwasserstoffe müssen mindestens eine Mehrfachbindung aufweisen. Bevorzugt sind Kohlenwasserstoffe mit mindestens einer olefinischen Doppelbindung, besonders bevorzugt sind Kohlenwasserstoffe mit einer olefinischen Doppelbindung. Grundsätzlich ist die Anzahl der Kohlenstoffatome von Verbindung, die mindestens eine Mehrfachbindung, vorzugsweise mindestens eine olefinische Doppelbindung aufweisen, nicht begrenzt. Technische relevant sind aber nur die Carbonylierung von C2- bis C20-Kohlenwasserstoffen mit mindestens einer Mehrfachbindung, vorzugsweise mindestens einer olefinischen Doppelbindung. In einer bevorzugten Ausführungsform der vorliegenden Erfindung können C3- bis C16-Kohlenwasserstoffe, besonders bevorzugt C4- bis C12-Kohlenwasserstoffe mit mindestens einer Mehrfachbindung, vorzugsweise mindestens einer olefinischen Doppelbindung eingesetzt werden. Darunter fallen insbesondere n-Alkene, iso-Alkene, Cycloalkene und aromatische Alkene mit 2 bis 20 Kohlenstoffatomen, vorzugsweise 3 bis 16 Kohlenstoffatomen, besonders bevorzugt 4 bis 12 Kohlenstoffatomen.

Die vorgenannt beschriebenen Kohlenwasserstoffe können zusätzlich zu der mindestens einen olefinischen Doppelbindungen eine oder mehrere weitere funktionelle Gruppen enthalten. Beispiele für geeignete funktionelle Gruppen sind Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl-, Amino-, Ether-, Thioether-, Aryl-, Heteroaryl- oder Silylgruppen und/oder Halogensubstituenten.

Besonders bevorzugte Kohlenwasserstoffe, die in Schritt a) des erfindungsgemäßen Verfahrens eingesetzt werden, weisen nur eine olefinische Doppelbindung auf, insbesondere n-Alkene und iso-Alkene mit 2 bis 20 Kohlenstoffatomen, vorzugsweise 3 bis 16 Kohlenstoffatomen, besonders bevorzugt 4 bis 12 Kohlenstoffatomen. Die eingesetzten Kohlenwasserstoffe sind vorzugsweise unsubtituiert.

Die eingesetzten und vorher beschriebenen Kohlenwasserstoffe werden erfindungsgemäß mit Kohlenmonoxid (CO) und einem Alkohol zum entsprechenden Ester in Schritt a) umgesetzt. Das Kohlenmonoxid kann dabei direkt als Einsatzgemisch oder durch die Zugabe eines Kohlenmonoxid-haltigen Gases, ausgewählt aus Synthesegas, Wassergas, Generatorgas und anderen Kohlenmonoxid-haltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei der Umsetzung in Schritt a) eingesetzte Alkohol ist ein Mono- oder Polyalkohol (zwei oder mehr OH-Gruppen) mit 1 bis 50 Kohlenstoffatomen, vorzugsweise 1 bis 15 Kohlenstoffatomen, besonders bevorzugt 1 bis 10 Kohlenstoffatomen oder eine Mischung aus zwei oder mehr Mono- und/oder Polyalkoholen. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol oder Mischungen daraus, vorzugsweise Ethanol, 1-Propanol, 1-Butanol, 1-Pentanol, 1-Hexanol, 2-Propanol, tert-Butanol, 3-Pentanol, 2-Propylheptanol.

Der in Schritt a) eingesetzte Alkohol wird vorzugsweise in einem Molverhältnis zum eingesetzten Kohlenwasserstoff (Alkohol: Kohlenwasserstoff) von 2 bis 20, weiterhin bevorzugt 3 bis 10 und besonders bevorzugt 4 bis 6 eingesetzt.. Der Monoalkohol wird somit - bezogen auf den eingesetzten Kohlenwasserstoff - im molaren Überschuss hinzugefügt. Dadurch kann der Alkohol sowohl als Edukt für die Carbonylierung als auch als Lösemittel fungieren. Der in Schritt a) eingesetzte Alkohol wird, wenn es sich um einen Polyalkohol handelt, in einem Molverhältnis zum eingesetzten Kohlenwasserstoff (Kohlenwasserstoff: Polyalkohol) von 2 bis 20, vorzugsweise von 3 bis 10 und besonders bevorzugt von 4 bis 8 eingesetzt. Der Polyalkohol wird bezogen auf den eingesetzten Kohlenwasserstoff also im molaren Unterschuss hinzugefügt.

Die erfindungsgemäße Umsetzung in Schritt a) wird in Gegenwart eines homogenen Katalysatorsystems durchgeführt. Bevorzugt umfasst das homogene Katalysatorsystem zumindest ein Metall aus der Gruppe 8 bis 10 des Periodensystems der Elemente (PSE) oder eine Verbindung davon, einen phosphorhaltigen Liganden und eine Säure als Co-Katalysator.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [$PdCl_2$], Palladium(II)-Acetylacetonat [$Pd(acac)_2$], Palladium(II)-Acetat [$Pd(OAc)_2$], Dichloro-(1,5-cyclooctadien)palladium(II) [$Pd(cod)Cl_2$], Bis(dibenzylideneaceton)palladium(0) [$Pd(dba)_2$], Tris(dibenzylideneaceton)dipalladium(0) [$Pd_2(dba)_3$] Bis(acetonitril)-dichloropalladium(II) [$Pd(CH_3CN)_2Cl_2$], Palladium(cinnamyl)-dichlorid [$Pd(cinnamyl)Cl_2$]. Vorzugsweise kommen die Verbindungen [$Pd(acaC)_2$] oder [$Pd(OAC)_2$] zum Einsatz. Die Metallkonzentration von Palladium in Schritt a) beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für die das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann für die beschriebene Umsetzung in Schritt a) 1 : 1 bis 10 : 1, vorzugsweise 2 : 1 bis 6 : 1, besonders bevorzugt 3 : 1 bis 5 : 1 betragen.

Das homogene Katalysatorsystem umfasst weiterhin eine Säure, wobei es sich insbesondere um eine Brönsted- oder eine Lewis-Säure handelt. Als Lewis-Säure können insbesondere Lewis Säuren mit einem LAU-Wert von mehr als 25, vorzugsweise mit einem LAU-Wert von 29. Der LAU-Wert ist eine Methode zur Bestimmung der Stärke von Lewis-Säuren (JR Gaffen et al., Chem, Vol. 5, Issue 6, S. 1567-1583). Als Lewis-Säure werden vorzugsweise Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs $\leq$ 5, besonders bevorzugt eine Säurestärke von pKs $\leq$ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 4 : 1 hinzugeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure.

Die Umsetzung bzw. Carbonylierung des eingesetzten Kohlenwasserstoffs mit olefinischer Doppelbindung in Schritt a) wird vorzugsweise bei einer Temperatur von 25 bis 140 °C, weiterhin bevorzugt bei einer Temperatur von 70 bis 130 °C und besonders bevorzugt bei einer Temperatur von 80 bis 120 °C durchgeführt. Der Druck in Schritt a) kann zwischen 5 und 60 bar, vorzugsweise zwischen 10 und 40 bar, besonders bevorzugt zwischen 15 und 30 bar betragen.

Die beschriebene Umsetzung in Schritt a) findet in einer geeigneten Reaktionszone statt. Die Reaktionszone für die Umsetzung umfasst mindestens einen Reaktor, kann aber auch aus zwei oder mehr Reaktoren bestehen. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein.

Durch die oben beschriebene Umsetzung in Schritt a) wird ein flüssiges Produktgemisch erhalten, die zumindest den durch die Umsetzung gebildeten Ester, das homogene Katalysatorsystem, nicht umgesetzte Alkohole A, und ggf. weitere Komponenten wie Leichtsieder, beispielsweise leichtsiedende Nebenprodukte wie Ether, und/oder Hochsieder und/oder nicht umgesetzte Kohlenwasserstoffe umfasst. Das Produktgemisch wird dann der nachfolgenden Membrantrennung in Schritt b) zugeführt. Bei der Umsetzung in Schritt a) kann zudem ein Abgas, welches zumindest aus unreaktiven Verunreinigungen wie Stickstoff, Wasserstoff und leichtsiedenden Nebenprodukten (beispielsweise die bereits genannten Ether) aus der Reaktionszone entnommen werden. Die Verunreinigungen und leichtsiedenden Nebenprodukte könnten sich akkumulieren und den Partialdruck des Reaktionsgases (CO) auf lange Sicht erniedrigen, wodurch die Reaktion verlangsamt werden könnte.

Im nachfolgenden Schritt b) wird das flüssige Produktgemisch einer Membrantrennung zugeführt, um das homogene Katalysatorsystem vom Produktgemisch abzutrennen. Durch das erfindungsgemäße Membranmaterial werden das homogene Katalysatorsystem und nicht umgesetzter Kohlenwasserstoff und/oder nicht umgesetzter Alkohol im Retentat angereichert, während der in Schritt a) gebildete Ester im Permeat angereichert wird. Das Permeat, welches an gebildeten Ester angereichert ist, wird dann zum nachfolgenden Schritt c) geführt. Das Retentat, welches das angereicherte homogene Katalysatorsystem umfasst, wird dann in die Reaktionszone zurückgeführt. Bei der Rückführung des Retentats kann weiterhin ein Purgestrom, welcher inerte Alkane, leichtsiedende Nebenprodukte (bspw. Ether), mögliche Abbauprodukte des Katalysatorsystems oder andere Verunreinigungen enthalten kann, die durch die eingesetzten Kohlenwasserstoffströme eingetragen werden, wie beispielsweise Spuren von Wasser oder Stickstoff, entnommen werden, um eine Akkumulation in der oder den Reaktionszonen zu vermeiden. Die Rückführung des Retentats sorgt dafür, dass das bei der Membrantrennung im Retentat angereicherte Katalysatorsystem zur Reaktion zurückgeführt wird. Katalysatorverluste durch im Permeat befindliche Reste an Katalysator, die durch eine Membran durchtreten, sind meistens nicht ganz zu vermeiden, aber die erwähnte Verringerung der Verluste führt dazu, dass weniger Katalysator durch Zuführung von frischem Katalysator ersetzt werden muss.

Die Membrantrennung beruht auf der Semipermeabilität der dabei eingesetzten Membran, welche für bestimmte Stoffe durchlässig und für andere Stoffe undurchlässig oder deutlich weniger durchlässig ist. Die in Schritt b) des erfindungsgemäßen Verfahrens eingesetzte Membran umfasst eine auf einer Unterstruktur aufgebrachte trennaktive Schicht, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht. In einer bevorzugten Ausführungsform der vorliegenden Erfindung besteht die bei der Membrantrennung in Schritt b) eingesetzte Membran aus einer auf einer Unterstruktur aufgebrachte trennaktive Schicht, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht.

Die Permeanz (Engl.: permeance) ist das wesentliche Unterscheidungsmerkmal zwischen der trennaktiven Schicht, an der bzw. in der die gewünschte Trennung abläuft, und der Unterstruktur, die keine Trennwirkung entfalten soll. Mit Permeanz ist im Rahmen der vorliegenden Erfindung der Druck-normalisierte Fluss (Engl.: pressure-normalized flux) gemeint. Für Membranen, welche in der Gastrennung eingesetzt werden, ist für Permeanz eine häufig genutzte Einheit gpu (Engl.: Gas Permeation Units). Für Membranen im Bereich der organophilen Nanofiltration (Engl.: Organic Solvent Nanofiltration) hat sich die Einheit L $m^{-2}$ $h^{-1}$ $bar^{-1}$ bewährt. Die Permeanz ist von der Permeabilität zu unterscheiden, da Permeabilität neben Druck auch in Bezug auf die Schichtdicke normalisiert (Engl.: thickness-normalized) ist. Es ist erfindungsgemäß bevorzugt, wenn die Unterstruktur keinen nennenswerten Transportwiderstand gegenüber der zu trennenden Mischung bietet und daher eine mindestens zweifach höhere Permeanz, vorzugsweise eine mindestens 10-fach höhere Permeanz, besonders bevorzugt eine mindestens 100-fach höhere Permeanz aufweist als die trennaktive Schicht.

Zur Messung der Permeanz von Unterstrukturen und trennaktiven Schichten eignet sich z.B. eine Messung der Luftdurchlässigkeit nach DIN EN ISO 9073-15 (August 2008). Die Messung von Luftdurchlässigkeit nach diesem Verfahren findet aufgrund der hohen Durchlässigkeit bei Differenzdrücken von nur etwa 2 mbar statt. Gängige Membranen für den Einsatz in der organophilen Nanofiltration haben in Lösemitteln wie Aceton oder Toluol eine Permeanz im Bereich von etwa 0,1 bis 10 L $m^{-2}$ $h^{-1}$ $bar^{-1}$. Nanofiltrationsmembranen werden daher typischerweise bei Differenzdrücken zwischen 20 bis 40 bar (20,000 bis 40,000 mbar) im Temperaturbereich leicht über Raumtemperatur (z.B. 30 bis 40 °C) vermessen, entweder als Flachmembranen mit aktiven Membranflächen von ca. 80 $cm^2$ oder als kleine Spiralwickelelemente mit aktiven Membranflächen von 0,1 bis 2 $m^2$. Die Permeanz einer PEEK-Membran im System 43 : 57 w/w Methanol: Methyloctanoat bei 20 bar Transmembrandruck und 40 °C ist zum Beispiel etwa 0,2 L $m^{-2}$ $h^{-1}$ $bar^{-1}$.

Die in Schritt b) eingesetzte Membran umfassend oder bestehend aus trennaktive(r) Schicht und Unterstruktur ist vorzugsweise säurestabil. Der Begriff "säurestabil" meint im Rahmen der vorliegenden Erfindung, dass die Membran mindestens 300 h in Anwesenheit der Säure des Katalysatorsystems, insbesondere einer Brönsted-Säure mit einem $pK_S \leq 5$, besonders bevorzugt mit einem $pK_S \leq 3$ oder einer Lewis-Säure mit einem LAU-Wert von mehr als 25, vorzugsweise mit einem LAU-Wert von 29, stabil ist und nicht zerstört wird, d. h. die trennaktive Schicht und die Unterstruktur bleiben intakt.

Die trennaktive Schicht der Membran besteht aus einem Polyaryletherketon (PAEK). PAEK zeichnet sich dadurch aus, dass innerhalb der Wiederholungseinheit Arylgruppen über mindestens eine Etherfunktionalität und mindestens eine Ketonfunktionalität verknüpft sind. Eine erfindungsgemäß bevorzugte trennaktive Schicht besteht aus einem Polyetheretherketon (PEEK). Es hat sich gezeigt, dass diese Materialien chemisch besonders stabil sind, insbesondere auch gegenüber Säuren. Als trennaktive Schicht wird weiterhin bevorzugt ein Polyaryletherketon (PAEK) mit einem Sulfonierungsgrad von weniger als 20%, besonders bevorzugt von weniger als 10%, besonders bevorzugt Polyetheretherketon (PEEK) mit einem Sulfonierungsgrad von weniger als 20%, besonders bevorzugt von weniger als 10% eingesetzt. Der Sulfonierungsgrad kann darüber bestimmt werden, dass zunächst die Gehalte von Kohlenstoff (C) und Schwefel (S) bestimmt werden. Dies kann beispielsweise mittels Elementaranalyse oder für Schwefel auch mittels Titration erfolgen. Der Sulfonierungsgrad kann dann anhand der folgenden Formel berechnet werden:

$$Sulfonierungsgrad\ (\%) = \frac{experimentell\ bestimmtes\ Verhältnis\ von\ S\ zu\ C\ in\ der\ Probe}{theor.\ Verhältnis\ von\ S\ zu\ C\ bei\ 100\%iger\ Sulfonierung} * 100$$

Es ist möglich, dass das für die Herstellung eingesetzte PAEK-Rohpolymer schon einen geringen Anteil an Schwefel enthalten kann, was einem Sulfonierungsgrad von bis zu 3 % entsprechen kann. Dieser geringe Anteil an Schwefel kann z. B. aus dem Einsatz des Hochtemperaturlösemittels Diphenylsulfon bei der Herstellung resultieren und entspricht nicht zwangsläufig einer Sulfonierung. Es kann daher in bestimmten Fällen notwendig sein, den Schwefelgehalt des Rohpolymers vor Verarbeitung zur Membran kennen und zu berücksichtigen, um den Sulfonierungsgrad der resultierenden Membran korrekt zu bestimmen.

Die Unterstruktur der Membran enthält erfindungsgemäß ein Polyaryletherketon (PAEK), vorzugsweise aus einem Polyetheretherketon (PEEK). Im Gegensatz zur trennaktiven Schicht kann die Unterstruktur auch andere Komponenten, beispielsweise andere Polymere enthalten. Es ist erfindungsgemäß jedoch bevorzugt, wenn das Polyaryletherketon (PAEK), vorzugsweise das Polyetheretherketon (PEEK) die Hauptkomponente ist, d. h. die Unterstruktur zu mehr als 50 Gew.- % aus dem Polyaryletherketon (PAEK) besteht, vorzugsweise dem Polyetheretherketon (PEEK). In einer bevorzugten Ausführungsform der vorliegenden Erfindung besteht die Unterstruktur zu mindestens 90 Gew.-% aus dem Polyaryletherketon (PAEK), vorzugsweise dem Polyetheretherketon (PEEK), weiterhin bevorzugt zu mindestens 99 Gew.-% aus dem Polyaryletherketon (PAEK), vorzugsweise dem Polyetheretherketon (PEEK). In einer besonders bevorzugten Ausführungsform besteht die Unterstruktur ausschließlich aus dem Polyaryletherketon (PAEK), vorzugsweise dem Polyetheretherketon (PEEK). Es hat sich gezeigt, dass diese Materialien chemisch besonders stabil sind, insbesondere auch gegenüber Säuren.

Die Unterstruktur kann dabei aus mehreren Schichten bestehen, wobei die einzelnen Schichten aus unterschiedlichen Materialien, z.B. unterschiedlichen Polymeren, Mischungen aus unterschiedlichen Materialien, oder Verbundsystemen bestehen können, wobei die vorgenannten bevorzugten Mindestmengen an Polyaryletherketon (PAEK), vorzugsweise dem Polyetheretherketon (PEEK) vorhanden sein müssen. Im einfachsten Fall besteht die Unterstruktur aus einer einzigen Schicht. Ein Beispiel dafür sind Vliesstoffe, insbesondere des Typs Viledon® Novatexx 2471 (Freudenberg Filtration Technologies, Germany). Viledon® Novatexx 2471 besteht aus Mikrofasern, die im Kern aus Polypropylen (PP) und im Mantel aus Polyethylen (PE) bestehen.

Die erfindungsgemäße Membran ist vorzugsweise eine Nanofiltrationsmembran, welche bei 40 °C eine Permeanz für einfache aromatische Kohlenwasserstoffe, bspw. Toluol, von 0,01 bis 10 L/(m$^2$ h bar), sowie einen Molecular Weight Cutoff (MWCO) zwischen 100 bis 2000 Da aufweist. Der MWCO ist als das Molekulargewicht einer Komponente mit einem Rückhalt durch die Membran von 90% definiert. Eine Nanofiltrationsmembran ist gemäß der vorliegenden Erfindung eine Membran, welche für Moleküle mit einem Molekülgewicht im Bereich zwischen 200 bis 2000 Da eine geeignete Trennwirkung entfaltet.

Ein Verfahren zur Herstellung der bei der Membrantrennung in Schritt b) eingesetzten Membran umfasst zumindest die folgenden Schritte:

a) Herstellen einer Polymerlösung, umfassend ein Polyaryletherketon (PAEK), vorzugsweise ein Polyetheretherketon (PEEK), und mindestens ein Lösemittel;

b) Bereitstellen einer Unterstruktur, die ein Polyaryletherketon (PAEK) enthält und die eine höhere Permeanz aufweist als die nachfolgend gebildete trennaktive Schicht;

c) Auftragen der Polymerlösung auf die Unterstruktur;

d) Durchführen einer Phaseninversion durch Eintauchen der Unterstruktur, auf die in Schritt c) die Polymerlösung aufgetragen worden ist, in einer Flüssigkeit, vorzugsweise einer wässrigen Flüssigkeit, wodurch sich die trennaktive Schicht ausbildet.

In Schritt a) des Verfahrens wird zunächst ein Polymerlösung hergestellt, die dann nachfolgend auf die Unterstruktur aufgetragen werden kann. Als Polymer wird entsprechend ein Polyaryletherketon (PAEK), vorzugsweise ein Polyetheretherketon (PEEK) oder ein Polyetherketonketon (PEKK). Hier können kommerziell verfügbare Rohstoffe eingesetzt werden, beispielsweise das Polyetheretherketon (PEEK) Vestakeep® 4000 von der Evonik Industries AG, KetaSpire® von der Solvay GmbH oder PEEK 450G™ von Victrex™. Die Rohpolymere können vor Einsatz in a) optional in der Schmelze filtriert werden, um z. B. Stippen, Gele oder andere Verunreinigungen zu entfernen. Es eignet sich z. B. eine Tiefenfiltration bei Temperaturen oberhalb von 350 °C mit Filtern mit Filterfeinheiten von 10 bis 50 μm.

Als Lösemittel kann jedes Lösemittel eingesetzt werden, in dem sich das eingesetzte Polyaryletherketon (PAEK) oder das eingesetzte Polyetheretherketon (PEEK) lösen. Bevorzugt wird im Rahmen der vorliegenden Erfindung eine Säure oder eine Mischung von zwei oder mehr Säuren eingesetzt. In einer weiterhin bevorzugten Ausführungsform der vorliegenden Erfindung wird als Lösemittel in Schritt a) Methansulfonsäure, Schwefelsäure oder eine Mischung aus Methansulfonsäure und Schwefelsäure eingesetzt. Ganz besonders bevorzugt wird als Lösemittel eine Mischung aus Methansulfonsäure und Schwefelsäure eingesetzt, wobei das Masseverhältnis Methansulfonsäure : Schwefelsäure im Bereich von 6 : 1 bis 1 : 1 liegt. Die hieraus resultierenden Polymerlösungen weisen vorzugsweise Viskositäten zwischen 1,000 mPa·s und 200,000 mPa·s auf, besonders bevorzugt zwischen 5,000 mPa·s und 100,000 mPa·s. Die Polymerlösung kann vor dem Einsatz den nachfolgenden Schritten optional filtriert werden, um z. B. Stippen, Gele oder andere Verunreinigungen zu entfernen. Die Polymerlösung kann vor dem Einsatz den nachfolgenden Schritten auch entgast werden, um Luftblasen zu entfernen.

In Schritt b) wird eine Unterstruktur bereitgestellt, die ein Polyaryletherketon (PAEK), vorzugsweise ein Polyetheretherketon (PEEK) enthält und die eine höhere Permeanz aufweist als die nachfolgend gebildete trennaktive Schicht. Die Unterstruktur weist insbesondere eine mindestens zweifach höhere Permeanz, vorzugsweise eine mindestens 10-fach höhere Permeanz, besonders bevorzugt eine mindestens 100-fach höhere Permeanz auf als die nachfolgend gebildete trennaktive Schicht. Entsprechende Unterstrukturen sind dem Fachmann geläufig. Da die Unterstruktur eine stabilisierende Funktion ausübt ist nur wichtig, dass sie eine höhere Permeanz aufweist und somit keinen wesentlichen Massentransferwiderstand ausübt. Strukturell sind die als Unterstruktur einsetzbaren Materialien also kaum beschränkt solange sie eine ausreichende mechanische Stabilität gewährleisten.

Ebenso wichtig und vorteilhaft ist, dass die Unterstruktur ein Polyaryletherketon (PAEK), vorzugsweise ein Polyetheretherketon (PEEK) enthält. Es ist erfindungsgemäß bevorzugt, dass das Polyaryletherketon (PAEK), vorzugsweise das Polyetheretherketon (PEEK) in einem hohen Anteil enthalten ist. Erfindungsgemäß bevorzugt besteht die Unterstruktur zu mindestens 90 Gew.-% aus dem Polyaryletherketon (PAEK) bzw. dem Polyetheretherketon (PEEK), vorzugsweise zu mindestens 99 Gew.-% aus dem Polyaryletherketon (PAEK) bzw. dem Polyetheretherketon (PEEK) und besonders bevorzugt ausschließlich aus dem Polyaryletherketon (PAEK) bzw. dem Polyetheretherketon (PEEK).

Im nachfolgenden Schritt c) wird dann die in Schritt a) hergestellte Polymerlösung auf die in Schritt b) bereitgestellte Unterstruktur aufgetragen. Das Auftragen kann grundsätzlich manuell mittels eines Rakels erfolgen. Industrielle Verfahren zum Auftragen entsprechender Polymerlösung sind beispielsweise der Walzenauftrag (3- bis 5-Walzensysteme), die Commabar, Schlitzdüsensysteme, z. B. Breitschlitzdüsen oder automatisierte Rakelsysteme, über die die Polymerlösung kontinuierlich aufgetragen werden kann. Diese und anderer Verfahren sind dem Fachmann aber grundsätzlich bekannt. Bevorzugt im Rahmen der vorliegenden Erfindung wird die Polymerlösung mittels eines Rakels aufgetragen, weil dies eine einfachere Handhabung und Reinigung ermöglicht.

Nach dem Auftragen der Polymerlösung in Schritt c) wird die Phaseninversion in Schritt d) durchgeführt. Dazu wird die Unterstruktur, auf die in Schritt c) die Polymerlösung aufgetragen worden ist, in eine Flüssigkeit eingetaucht, wodurch sich die trennaktive Schicht ausbildet. Hierbei wird das Lösungsmittel der Polymerlösung aus dem aufgetragenen Film verdrängt und durch die Flüssigkeit verdünnt. Das gelöste Polymer ist entgegen dem Lösungsmittel nicht in der Flüssigkeit löslich, sodass dieses auf der Unterstruktur ausfällt und die trennaktive Schicht bildet. Als Flüssigkeit kann grundsätzlich jede Flüssigkeit eingesetzt werden, durch die das Lösungsmittel verdrängt und das Polymer unlöslich ist. Vorzugsweise wird als Flüssigkeit in Schritt d) eine wässrige Flüssigkeit, ein protisches Lösemittel wie Methanol oder eine Mischung dieser eingesetzt. Ferner können der Flüssigkeit lösliche Additive zugesetzt werden, welche den Vorgang der Phaseninversion günstig beeinflussen, indem sie diesen zum Beispiel verlangsamen. Entsprechende Additive sind dem Fachmann bekannt. Besonders bevorzugt wird in Schritt d) Wasser, weiterhin bevorzugt vollentsalztes Wasser eingesetzt.

Bei Herstellung von Membranen per Phaseninversion kann zwischen symmetrischen und asymmetischen (integrally skinned asymmetric) Membranen unterschieden werden. Entsteht symmetrische Membran, weist diese Membran eine durchgehend homogene Struktur auf. Entsteht eine asymmetrische Membran, weist diese Membran ausgehend von der Feedseite (Seite, auf die der Feed auf die Membran bzw. die trennaktive Schicht trifft), einen oberen Teil mit dichter Struktur und einen darunterliegenden unteren Teil mit einer fingerähnlichen Struktur auf. Im Fall der asymmetrischen Membran ist oft nur der obere dichte Teil trennaktiv. Der untere fingerähnliche Teil wird im Rahmen der vorliegenden Erfindung trotzdem der trennaktiven Schicht zugerechnet. Im Falle einer asymmetrischen Membran entstehen nämlich die trennaktive Schicht und die fingerähnliche Struktur aus derselben Polymerlösung und sind somit aus demselben Polymer, wenn sich auch aufgrund der Phaseninversionskinetik Unterschiede in Bezug auf die physische Beschaffenheit, z.B. Dichte oder Kristallinität, ergeben können.

In einer bevorzugten Ausführungsform der vorliegenden Erfindung umfasst das Verfahren zur Herstellung der Membran den folgenden zusätzlichen Schritt:

e) Waschen der in Schritt d) hergestellten Membran zur Entfernung der restlichen Säure.

Das Waschen ist deshalb optional, weil es durchaus denkbar ist, dass die Membran auch ohne vorheriges Waschen eingesetzt wird. Letztendlich kommt es auf die spezifische Anwendung an. Das Waschen dient in erster Linie der Entfernung von noch aus der Herstellung enthaltenen Säure. Sofern die in Schritt d) hergestellte Membran gemäß Schritt e) gewaschen wird, kann dazu eine wässrige Flüssigkeit oder ein anderes geeignetes Lösemittel eingesetzt werden. In einer bevorzugten Ausführungsform der vorliegenden Erfindung enthält die Membran nach dem Waschen weniger als 20% der bei der Herstellung eingesetzten Säure, vorzugsweise weniger als 10% der bei der Herstellung eingesetzten Säure, besonders bevorzugt weniger als 5% der bei der Herstellung eingesetzten Säure in Bezug auf die Gesamtmenge der Säure in der zur Herstellung der Membran eingesetzten Polymerlösung. Die wässrige Flüssigkeit für das Waschen in Schritt e) kann beispielsweise Wasser sein, welches zusätzlich Additive enthält, beispielsweise Alkohole oder Salze bzw. Elektrolyte. Bevorzugt wird zum Waschen eine wässrige Flüssigkeit, besonders bevorzugt vollentsalztes Wasser verwendet.

Nach dem Waschen kann ein Flüssigkeitsaustausch durchgeführt werden, wobei die wässrige Flüssigkeit durch eine andere Flüssigkeit, vorzugsweise ein organisches Lösemittel ausgetauscht wird. Das organische Lösemittel wird vorzugsweise aus der Gruppe, bestehend aus Methanol, Ethanol, Isopropanol, Aceton, THF und n-Hexan, ausgewählt. Bevorzugte organische Lösemittel aus dieser Gruppe sind Ethanol, Isopropanol und Aceton.

In einer besonders bevorzugten Ausführungsform der vorliegenden Erfindung umfasst das Verfahren zur Herstellung der erfindungsgemäßen Membranen den folgenden zusätzlichen Schritt:

f) Trocknen der aus Schritt d) oder Schritt e) erhaltenen Membran bei einer Temperatur im Bereich von 80 bis 180 °C, bevorzugt bei 110 bis 160 °C.

Das Trocknen kann auch ohne vorheriges Waschen stattfinden, beispielsweise dann, wenn der pH-Wert beim Einsatz der Membran nicht neutral ist. Das Trocknen der erhaltenen Membran führt dazu, dass noch vorhandene Flüssigkeit zumindest teilweise aus der Membran ausgetrieben wird. Die Permeanz der Membran wird deutlich geringer, wodurch die Membran ihre Eigenschaft als Nanofiltrationsmembran erhält. Bei der Trocknung ergibt sich bei der Verwendung der erfindungsgemäßen Unterstruktur zudem der Vorteil, dass die erfindungsgemäße Unterstruktur, die ein Polyaryletherketon (PAEK), vorzugsweise ein Polyetheretherketon (PEEK) enthält, im Gegensatz z. B. zu Polypropylen nicht schon bei ca. 120 bis 140 °C anfängt zu schmelzen. Dadurch können höhere Temperaturen eingesetzt werden und kürze Trockenzeiten realisiert werden. Außerdem hat das beispielhaft erwähnte Polypropylen den Nachteil, dass es auch weit unterhalb des Schmelzpunktes zur Kaltverformung neigt. Das bedeutet, dass Polypropylen sich auch bei Temperaturen verformen und es dadurch zu Problemen kommen kann. So müssen zum Beispiel im Falle einer Unterstruktur aus Polypropylen in einem kontinuierlichen Prozess von Rolle zu Rolle die Lenkwalzen penibel ausgerichtet werden, um eine Verstreckung der Unterstruktur und somit typischerweise auch der aufgetragenen Membran zu vermeiden.

Die erwähnte Trocknung im optionalen Schritt f) des erfindungsgemäßen Verfahrens kann kontinuierlich, beispielsweise in einem Bandtrockner, oder diskontinuierliche, beispielsweise in einem geeigneten Ofen, erfolgen. Die Trocknung kann dabei bei atmosphärischem Druck (gilt für kontinuierliche oder diskontinuierliche Trocknungsverfahren) oder bei Vakuum in einem Bereich von 10 mbar bis 300 mbar, vorzugsweise 50 mbar bis 250 mbar (gilt nur für kontinuierliche Trocknungsverfahren) durchgeführt wird.

Die Membrantrennung in Schritt b) wird vorzugsweise Temperatur von 25 bis 100°C, weiterhin bevorzugt 30 bis 80°C und besonders bevorzugt 40 bis 70°C durchgeführt. Um das Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wodurch ein anderer Strom innerhalb des erfindungsgemäßen Verfahrens erhitzt wird. Optional ist auch ein Degasingschritt zwischen der Reaktionszone in Schritt a) und der Membrantrennung in Schritt b) vorhanden, um vorher leicht flüchtige Verbindungen wie Kohlenmonoxid und/oder restliche, über das Abgas nicht abgetrennte unreaktiven Verunreinigungen wie Stickstoff, Wasserstoff, Alkane und leichtsiedenden Nebenprodukte (beispielsweise die bereits genannten Ether) aus der Produktmischung zu entfernen. Dabei wird das Produktgemisch erst unter den Partialdruck der gelösten Komponenten wie Kohlenmonoxid entspannt, so dass diese ausgasen, um dann wieder den Druck zu erhöhen, der in der Membrantrennung vorgesehen ist.

Der Transmembrandruck (TMP) kann in Schritt b) 10 bis 60 bar, vorzugsweise 15 bis 55 bar, besonders bevorzugt 20 bis 50 bar betragen (relativ). Der permeatseitige Druck kann dabei oberhalb des atmosphärischen Drucks bis 15 bar, vorzugsweise 3 bis 7 bar betragen, woraus sich anhand des TMP dann der retentaseitige Druck ergibt. In einer bevorzugten Ausführungsform sollte bei den Druckverhältnissen und insbesondere beim permeatseitigen Druck darauf geachtet werden, dass der Druck in Abhängigkeit von dem eingesetzten Kohlenwasserstoff, dem eingesetzten Alkohol und der vorhandenen Temperatur eingestellt wird, um eine Verdampfung nach dem Durchtritt durch die Membran zu vermeiden, da dadurch die gesamte Fahrweise instabil werden könnte. Gleiches gilt grundsätzlich auch für gelöste Komponenten wie Kohlenmonoxid, die optional durch den bereits genannten Degasingschritt entfernt werden können.

Das Permeat aus der Membrantrennung (Schritt b)) wird im nachfolgenden Schritt c) einem Trennverfahren ausgewählt aus der Gruppe, bestehend aus einer thermischen Trennung, beispielsweise Destillation, einer Extraktion, einer Kristallisation oder einer weiteren Membrantrennung, unterworfen, um den in Schritt a) gebildeten Ester vom restlichen Permeat abzutrennen. Das Trennverfahren ist vorzugsweise eine Destillation. Die entsprechenden Verfahrensbedingungen sind dem Fachmann bekannt.

Es kann vorkommen, dass bei dem in Schritt c) verwendeten Trennverfahren, insbesondere bei der Destillation, nicht nur der gebildete Ester aus dem Permeat abgetrennt wird, sondern auch die möglicherweise entstandenen Hochsieder, beispielsweise hochsiedende Nebenprodukte, die bei der Umsetzung in Schritt a) anfallen können. Um diese Hochsieder zu entfernen kann das erfindungsgemäße Verfahren einen Aufreinigungsschritt aufweisen, nämlich die Aufreinigung des gebildeten Esters durch Abtrennung des Esters von im Permeat enthaltenden Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Das in Schritt c) erhaltene zu großen Teilen vom in Schritt a) gebildeten Ester befreite Permeat, welches mindestens nicht umgesetzte Alkohole und/oder nicht umgesetzte Kohlenwasserstoffe enthält, wird in einer bevorzugten Ausführungsform einer Recyclekomponentenabtrennung unterworfen. Dabei werden die nicht umgesetzten Alkohole und/oder nicht umgesetzten Kohlenwasserstoffe vom restlichen Permeat, insbesondere den dort enthaltenen Leichtsiedern, mittels thermischer Trennung, Extraktion, Kristallisation oder Membrantrennung abgetrennt. Bevorzugt wird ein thermisches Trennverfahren, besonders bevorzugt eine weitere Destillation eingesetzt, um die nicht umgesetzten Alkohole und/oder nicht umgesetzten Kohlenwasserstoffe vom restlichen Permeat abzutrennen. Die Verfahrensbedingungen sind dem Fachmann grundsätzlich bekannt. Die dabei erhaltenen nicht umgesetzten Alkohole und/oder die nicht umgesetzten Kohlenwasserstoffe können dann in die Reaktionszone zurückgeführt werden.

Der nach dem erfindungsgemäßen Verfahren gebildete Ester kann in zwei weiteren Verfahrensschritten c1) und c2) umgeestert werden. Dabei wird der Teil des Esters, der dem in Schritt a) eingesetzten ersten Alkohol A entspricht, durch einen zweiten Alkohol B ausgetauscht. Diese Umesterung wird nach dem oben genannten Schritt c), optional nach dem möglichen Aufreinigungsschritt, durchgeführt und umfasst die folgenden Schritte:

c1) Umestern des in Schritt a) gebildeten Esters mit einem zweiten Alkohol B, wobei dieser zweite Alkohol sich von dem im Schritt a) eingesetzten Alkohol A unterscheidet, in einer zweiten Reaktionszone unter Erhalt eines zweiten Produktgemischs, welches zumindest den Ester mit dem zweiten Alkohol B, den abgespaltenen ersten Alkohol A und nicht umgesetzten zweiten Alkohol B umfasst;

c2) Abtrennen des gebildeten Esters mit dem zweiten Alkohol B vom restlichen zweiten Produktgemisch und insbesondere vom abgespaltenen ersten Alkohol A durch thermische Trennung und/oder mittels Membrantrennung und Rückführung des abgespaltenen ersten Alkohols A in die Reaktionszone aus Schritt a), sowie Rückführung des nicht umgesetzten Alkohols B in die zweite Reaktionszone.

In Schritt c1) erfolgt die eigentliche Umeesterung, also die Abspaltung des eigentlich in Schritt a) angebundenen ersten Alkohols A und die Anbindung des zweiten Alkohols B. Dazu wird der in Schritt a) gebildete Ester in einer Reaktionszone mit einem zweiten Alkohol B, der sich vom ersten Alkohol A unterscheidet, umgesetzt. In einer besonders bevorzugten Ausführungsform ist der bei der Umesterung eingesetzte zweite Alkohol B im Vergleich zu dem in Schritt a) eingesetzten ersten Alkohol A ein höhersiedender Alkohol. Der zweite Alkohol B wird bei der Umesterung vorzugsweise im Überschuss zugegeben, um die Umesterungsreaktion zu begünstigen.

Der bei der Umesterung in Schritt c1) eingesetzte zweite Alkohol ist vorzugsweise ein Mono- oder Polyalkohol (zwei oder mehr OH-Gruppen) mit 1 bis 50 Kohlenstoffatomen, weiterhin bevorzugt mit 1 bis 15 Kohlenstoffatomen, besonders bevorzugt mit 1 bis 10 Kohlenstoffatomen oder eine Mischung aus zwei oder mehr Mono- und/oder Polyalkoholen, mit der Maßgabe, dass der in Schritt a) eingesetzte erste Alkohol A und der zweite Alkohol B nicht identisch sind. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol, Pentaerythrit, Neopentylglykol, Trimethylolpropan, Dipentaerythritol oder Mischungen daraus, vorzugsweise Ethanol, 1-Propanol, 1-Butanol, 1-Pentanol, 1-Hexanol, 2-Propanol, tert-Butanol, 3-Pentanol, 2-Propylheptanol.

Die Umesterung in Schritt c1) wird vorzugsweise säure- oder basenkatalysiert durchgeführt. Als Säuren können Brönsted- oder Lewis-Säuren eingesetzt werden.

Geeignete Brönsted-Säuren für die Umesterung in Schritt c1) sind Perchlorsäure, Schwefelsäure, Phosphorsäure, Methylphosphonsäure oder eine Sulfonsäure, beispielsweise Methansulfonsäure, Trifluormethansulfonsäure, tert-Butansulfonsäure, p-Toluolsulfonsäure(pTSA), 2-Hydroxypropan-2-sulfonsäure, 2,4,6-Trimethylbenzolsulfonsäure oder Dodecylsulfonsäure. Vorzugsweise werden Schwefelsäure oder eine Sulfonsäure als Brönsted-Säure eingesetzt, besonders bevorzugt Schwefelsäure. Es können auch Metall oder deren Verbindungen eingesetzt werden, beispielsweise Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat sowie Natriummethanolat und Kaliummethanolat.

Geeignete Lewis-Säuren für die Umesterung in Schritt c1) sind Titan(IV)-isopropoxid, $Bu_2SnO$, BuSn(O)OH oder Aluminiumtriflat. Bevorzugt eingesetzte Lewis Säuren sind Titan(IV)-isopropoxid, und Aluminiumtriflat.

Geeignete Basen für die Umesterung in Schritt c1) sind Alkalimetalle, Alkalialkoxide, Alkali- oder Erdalkaliacetate oder -oxide, Alkali- oder Erdalkalialkoholate, wie NaEtOH oder MgEtOH oder Alkalicarbonate, wie $K_2CO_3$ oder $Cs_2CO_3$. Es können aber auch basische Ionenaustauscher oder NaOH eingesetzt werden. Bevorzugt werden Na- oder Mg-Alkoholate, wie NaEtOH oder MgEtOH, eingesetzt.

Die säurekatalysierten Umesterung wird vorzugsweise bei einer Temperatur von 60 bis 220 °C, weiterhin bevorzugt von 100 bis 210 °C und besonders bevorzugt bei 130 bis 200 °C durchgeführt. Die Reaktion findet vorzugsweise oberhalb des Siedepunktes des abzuspaltenden ersten Alkohols A statt, um den abgespaltenen ersten Alkohol A direkt aus dem Reaktionsgemisch zu entfernen und somit eine Gleichgewichtsverlagerung zur Produktseite zu begünstigen. Der zweite Alkohol B wird dabei vorzugsweise in einem signifikanten Überschuss, beispielsweise 30 : 1, zum in Schritt a) gebildeten Ester hinzugegeben.Die basenkatalysierte Umesterung findet vorzugsweise bei einer Temperatur von 20 bis 100 °C statt.

Durch die beschriebe Umesterung wird ein zweites Produktgemisch erhalten, welches zumindest den Ester mit dem zweiten Alkohol B, den abgespaltenen ersten Alkohol A und nicht umgesetzte zweite Alkohole B umfasst.

Der in Schritt c1) gebildete zweite Ester wird im nachfolgenden Schritt c2) vom restlichen zweiten Produktgemisch abgetrennt. Die Abtrennung wird mittels thermischer Trennung, vorzugsweise Destillation, und/oder mittels Membrantrennung, insbesondere mit den oben beschriebenen Membranmaterialien, durchgeführt. Die entsprechenden Verfahrensbedingungen sind dem Fachmann bekannt.

Es kann vorkommen, dass bei dem in Schritt c2) verwendeten Trennverfahren, insbesondere bei der Destillation, nicht nur der gebildete Ester vom restlichen zweiten Produktgemisch abgetrennt wird, sondern auch möglicherweise gebildete Hochsieder, beispielsweise hochsiedende Nebenprodukte, die bei der Umsetzung in Schritt c1) anfallen können. Um diese Hochsieder zu entfernen kann das erfindungsgemäße Verfahren einen Aufreinigungsschritt aufweisen, nämlich die Aufreinigung des in Schritt c1) gebildeten Esters durch Abtrennung des Esters von den vorhandenen Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Der in Schritt a) hergestellte und in Schritt c) aus dem Permeat abgetrennte und ggf. aufgereinigte Ester wird dann in Schritt d) einer Hydrolyse oder Verseifung unterworfen. Durch den dabei eingesetzten sauren Katalysator bzw. das dabei eingesetzte Verseifungsmittel wird die Estergruppe gespalten, wodurch eine Carbonsäure oder ein Carbonsäuresalz entsteht und der bei der Esterbildung in Schritt a) angebundenen Alkohol A bzw. den bei der Umesterung angebundenen Alkohol B zurückgewonnen werden kann. Es entsteht demnach bei der Verseifung ein Reaktionsgemisch, welches zumindest die Carbonsäure oder deren Salz, den Alkohol A oder B und nicht umgesetzte Ester umfasst.

Ohne Umesterung kann der mit der Hydrolyse oder Verseifung in Schritt d) zurückgewonnen Alkohol A in einem nachfolgenden Prozessschritt aus dem entstandenen Alkoholgemisch abgetrennt und in die erste Reaktionszone zurückgeführt werden. Wurde eine Umesterung durchgeführt und bei der Hydrolyse oder Verseifung in Schritt d) der Alkohol B zurückgewonnen, kann dieser Alkohol B im nachfolgenden Prozessschritt aus dem entstandenen Reaktionsgemisch abgetrennt und in die zweite Reaktionszone zurückgeführt werden.

Die Hydrolyse ist eine bekannte chemische Reaktion, bei der ein Ester mit Hilfe eines sauren Katalysators in eine Carbonsäure unter Abspaltung eines Alkohols überführt wird. Die typischen Bedingungen bei der Hydrolyse sind dem Fachmann bekannt.

Die Hydrolyse in Schritt d) findet in Anwesenheit eines sauren heterogenen oder homogenen Katalysators statt. Bekannte homogene Katalysatoren sind saure Verbindungen wie zum Beispiel Brönsted-Säuren, insbesondere HCl, $H_2SO_4$, Phosphorsäure, p-Toluolsulfonsäure und 4-Dodecylbenzolsulfonsäure, oder Lewis Säuren, insbesondere $AlCl_3$, ZnCl2, $HfCl_4 \cdot 2THF$, $Al(OTf)_3$. Bekannte heterogene Katalysatoren sind saure Verbindungen wie zum Beispiel Kationenaustauscher, saure H-Zeolithe, saure funktionalisierte Metalloxide mit Silica und Heteropolysäuren. Geeignete heterogene Katalysatoren sind insbesondere Amberlyst® 15, Amberlyte® IR 120 (H), $HClO_4$-$SiO_2$, 3-Propylsulfonsäure funktionalisierte Silica, Nafion®-$SiO_2$, Aciplex®-$SiO_2$, $Cs_{2.5}H_{0.5}PW_{12}O_{40}$, H-ZSM5, H-ZSM-5-C18, Z-beta-H-25, Z-beta-H-38, Z-beta-H-150, Z-beta-H-360, Z-Y-H-60, Z-Y-H-80.

Das Reaktionsgemisch aus der Hydrolyse in Schritt d) wird im nachfolgenden Schritt e) in mindestens einem Trennverfahrensschritt ausgewählt aus der Gruppe, bestehend aus einer thermischen Trennung, beispielsweise Destillation, einer Extraktion, einer Kristallisation oder einer weiteren Membrantrennung, unterworfen, um die in Schritt d) gebildete Carbonsäure oder deren Salz vom restlichen Reaktionsgemisch abzutrennen. Das Trennverfahren ist vorzugsweise eine Destillation. Die entsprechenden Verfahrensbedingungen sind dem Fachmann bekannt. Es kann auch eine mehrstufige Destillation durchgeführt werden.

Die Verseifung ist eine bekannte chemische Reaktion, bei der ein Ester mit Hilfe eines basischen oder enzymatischen Verseifungsmittels in ein Carbonsäuresalz unter Abspaltung eines Alkohols überführt wird. Die typischen Bedingungen bei der Verseifung sind dem Fachmann bekannt.

Die Verseifung in Schritt d) findet in Anwesenheit eines Verseifungsmittels statt. Bekannte Verseifungsmittel sind basische Verbindungen wie Kaliumhydroxid, Kalium(hydrogen)carbonat, Natriumhydroxid, Natrium(hydrogen)carbonat oder Aminverbindungen. Möglich ist auch eine Verseifung mit einem enzymatischen Verseifungsmittel, insbesondere Esterasen.

Das Reaktionsgemisch aus der Verseifung in Schritt d) wird im nachfolgenden Schritt e) in mindestens einem Trennverfahrensschritt ausgewählt aus der Gruppe, bestehend aus einer thermischen Trennung, beispielsweise Destillation, einer Extraktion, einer Kristallisation oder einer weiteren Membrantrennung, unterworfen, um die in Schritt d) gebildete Carbonsäure oder deren Salz vom restlichen Reaktionsgemisch abzutrennen. Das Trennverfahren ist vorzugsweise eine Destillation. Die entsprechenden Verfahrensbedingungen sind dem Fachmann bekannt. Es kann auch eine mehrstufige Destillation durchgeführt werden.

Zudem können in dem mindestens einen Trennverfahrensschritt auch die eingesetzten Alkohole A bzw. B abgetrennt und zur jeweiligen ersten oder zweiten Reaktionszone zurückgeführt werden. Bei der Rückführung kann ein Purgestrom entnommen werde, um beispielsweise Nebenprodukte aus dem Verfahren auszuschleusen.

Das Verfahren ist gemäß einer besonders bevorzugten Ausführungsform der vorliegenden Erfindung ein Verfahren zur Herstellung einer Carbonsäure oder deren Salz, wobei das Verfahren zur Herstellung Carbonsäure oder eines Carbonsäuresalzes die folgenden Schritte umfasst:

a) Herstellung eines Esters durch Umsetzen eines n-Alkens und/oder iso-Alkens mit 4 bis 12 Kohlenstoffatomen mit Kohlenmonoxid und mit einem Alkohol, ausgewählt aus der Gruppe, bestehend aus Methanol, Ethanol, 1-Propanol, 1-Butanol, 1-Pentanol, 1-Hexanol, 2-Propanol, tert-Butanol und 3-Pentanol, in Gegenwart eines homogenen Katalysatorsystems, welches Pd oder Pt oder eine Verbindung davon, einen phosphorhaltigen Liganden und eine Säure als Co-Katalysator umfasst, in einer Reaktionszone unter Erhalt eines Produktgemisches, wobei die Umsetzung bei einer Temperatur von 80 bis 120 °C und einem Druck von 10 bis 40 bar durchgeführt wird;

b) Durchführen einer Membrantrennung zur Abtrennung des homogenen Katalysatorsystems aus dem Produktgemisch, wodurch das homogene Katalysatorsystem und nicht umgesetzter Kohlenwasserstoff und/oder nicht umgesetzter Alkohol A, vorzugsweise nicht umgesetzter Alkohol A im Retentat angereichert werden und der in Schritt a) gebildete Ester im Permeat angereichert wird, wobei bei der Membrantrennung eine Membran eingesetzt wird, die eine auf einer Unterstruktur aufgebrachte trennaktive Schicht umfasst, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht;

c) Abtrennen des in Schritt a) gebildeten Esters aus dem Permeat in mindestens einem Trennverfahrensschritt, ausgewählt aus thermischer Trennung und Extraktion;

d) Verseifen des in Schritt a) hergestellten Esters in Anwesenheit eines Verseifungsmittels unter Erhalt eines Reaktionsgemisches, welches zumindest die Carbonsäure oder deren Salz, den abgespaltenen Alkohol A und nicht umgesetzte Ester umfasst;

e) Abtrennen der in Schritt d) gebildeten Carbonsäure oder deren Salz in mindestens einem Trennverfahrensschritt, ausgewählt aus thermischer Trennung und Extraktion.

## Patentansprüche

1. Verfahren zur Herstellung einer Carbonsäure oder deren Salz, wobei das Verfahren die folgenden Schritte umfasst:

a) Herstellung eines Esters durch Umsetzen (Carbonylieren) eines C2- bis C20-Kohlenwasserstoffs mit mindestens einer Mehrfachbindung, vorzugsweise mit mindestens einer olefinischen Doppelbindung mit Kohlenmonoxid und mit einem Alkohol A in Gegenwart eines homogenen Katalysatorsystems in einer Reaktionszone unter Erhalt eines Produktgemisches;

b) Durchführen einer Membrantrennung zur Abtrennung des homogenen Katalysatorsystems aus dem Produktgemisch, wodurch das homogene Katalysatorsystem und nicht umgesetzter Kohlenwasserstoff und/oder nicht umgesetzter Alkohol A, vorzugsweise nicht umgesetzter Alkohol A im Retentat angereichert werden und der in Schritt a) gebildete Ester im Permeat angereichert wird, wobei bei der Membrantrennung eine Membran eingesetzt wird, die eine auf einer Unterstruktur aufgebrachte trennaktive Schicht umfasst, wobei die trennaktive Schicht aus einem Polyaryletherketon (PAEK) besteht, die Unterstruktur ein Polyaryletherketon (PAEK) enthält und die Unterstruktur eine höhere Permeanz aufweist als die trennaktive Schicht;

c) Abtrennen des in Schritt a) gebildeten Esters aus dem Permeat in mindestens einem Trennverfahrensschritt, ausgewählt aus thermischer Trennung, beispielsweise Destillation, Extraktion, Kristallisation und Membrantrennung;

d) Hydrolyse oder Verseifen des in Schritt a) hergestellten Esters in Anwesenheit eines sauren Katalysators oder eines Verseifungsmittels unter Erhalt eines Reaktionsgemisches, welches zumindest die Carbonsäure oder deren Salz, den abgespaltenen Alkohol A, Wasser und nicht umgesetzte Ester umfasst;

e) Abtrennen der in Schritt d) gebildeten Carbonsäure oder deren Salz in mindestens einem Trennverfahrensschritt, ausgewählt aus thermischer Trennung, beispielsweise Destillation, Extraktion, Kristallisation und Membrantrennung.

2. Verfahren nach Anspruch 1, wobei die Unterstruktur der bei der Membrantrennung eingesetzten Membran zu mindestens 90 Gew.-% aus dem Polyaryletherketon (PAEK), vorzugsweise zu mindestens 99 Gew.-% aus dem Polyaryletherketon (PAEK) und besonders bevorzugt ausschließlich aus dem Polyaryletherketon (PAEK) besteht.

3. Verfahren nach Anspruch 2, wobei die Unterstruktur der bei der Membrantrennung eingesetzten Membran zu mindestens 90 Gew.-% aus einem Polyetheretherketon (PEEK), vorzugsweise zu mindestens 99 Gew.-% aus einem Polyetheretherketon (PEEK)und besonders bevorzugt ausschließlich aus dem einem Polyetheretherketon (PEEK) besteht.

4. Verfahren nach Anspruch 3, wobei die Unterstruktur der bei der Membrantrennung eingesetzten Membran ausschließlich aus einem Polyetheretherketon (PEEK) besteht.

5. Verfahren nach einem der vorhergehenden Ansprüche, wobei die trennaktive Schicht der bei der Membrantrennung eingesetzten Membran aus einem Polyetheretherketon (PEEK) besteht.

6. Verfahren nach Anspruch 1 oder 2, wobei die trennaktive Schicht und/oder die Unterstruktur aus einem Polyaryletherketon (PAEK) mit einem Sulfonierungsgrad von weniger als 20%, besonders bevorzugt weniger als 10% besteht.

7. Verfahren nach einem der Ansprüche 3 bis 5, wobei die trennaktive Schicht und/oder die Unterstruktur ein Polyetheretherketon (PEEK) mit einem Sulfonierungsgrad von weniger als 20%, besonders bevorzugt weniger als 10% umfassen oder daraus bestehen.

8. Verfahren nach einem der vorhergehenden Ansprüche, wobei die Unterstruktur der Membran eine mindestens zweifach höhere Permeanz, vorzugsweise eine mindestens 10-fach höhere Permeanz, besonders bevorzugt eine mindestens 100-fach höhere Permeanz aufweist als die trennaktive Schicht

9. Verfahren nach einem der vorhergehenden Ansprüche, wobei der Alkohol A ein Mono- oder Polyalkohol (zwei oder mehr OH-Gruppen) mit 1 bis 50 Kohlenstoffatomen, vorzugsweise 1 bis 15 Kohlenstoffatomen, besonders bevorzugt 1 bis 10 Kohlenstoffatomen oder eine Mischung aus zwei oder mehr Mono- und/oder Polyalkoholen ist.

10. Verfahren nach Anspruch 9, wobei der in Schritt a) eingesetzte Alkohol aus der Gruppe, bestehend aus Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol und Mischungen daraus, ausgewählt wird.

11. Verfahren nach einem der vorhergehenden Ansprüche, wobei das bei der Carbonylierung in Schritt a) eingesetzte homogene Katalysatorsystem zumindest ein Metall der Gruppe 8 bis 10 des Periodensystems der Elemente oder einer Verbindung davon, einen vorzugsweise phosphorhaltigen Liganden und eine Säure umfasst.

12. Verfahren nach Anspruch 11, wobei die Säure des Katalysatorsystems in Schritt a) eine Brönsted-Säure oder eine Lewis-Säure ist, wobei die Brönsted-Säure Perchlorsäure, Schwefelsäure, Phosphorsäure, Methylphosphonsäure oder eine Sulfonsäure ist und wobei die Lewis-Säure Aluminiumtriflat, Aluminiumchlorid, Aluminiumhydrid, Trimethylaluminium, Tris(pentafluorophenyl)boran, Bortrifluorid, Bortrichlorid oder eine Mischung davon ist.

13. Verfahren nach einem der vorhergehenden Ansprüche, wobei das Verfahren nach der Abtrennung in Schritt c) die folgenden weiteren Schritte aufweist:

c1) Umestern des in Schritt a) gebildeten Esters mit einem zweiten Alkohol, wobei dieser zweite Alkohol sich von dem im Schritt a) eingesetzten Alkohol unterscheidet, in einer zweiten Reaktionszone unter Erhalt eines zweiten Produktgemischs;
c2) Abtrennen des in Schritt c1) gebildeten Esters und Auftrennung des restlichen zweiten Produktgemisch durch thermische Trennung und/oder mittels Membrantrennung und
Rückführung des abgespaltenen ersten Alkohols in die erste Reaktionszone, sowie Rückführung des nicht umgesetzten zweiten Alkohols in die zweite Reaktionszone;
wobei der in Schritt c1) gebildete Ester in der Hydrolyse oder Verseifung in Schritt d) eingesetzt wird.

14. Verfahren nach Anspruch 13, wobei der in Schritt e) eingesetzte zweite Alkohol im Vergleich zum ersten Alkohol ein höhersiedender Alkohol ist.

15. Verfahren nach Anspruch 13 oder 14, wobei ein Teil der abgespaltenen ersten Alkohole bereits aus der zweiten Reaktionszone entnommen und zur ersten Reaktionszone zurückgeführt wird.

Europäisches
Patentamt
European
Patent Office
Office européen
des brevets

**EUROPÄISCHER RECHERCHENBERICHT**

**Nummer der Anmeldung**

**EP 21 21 5858**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 3 750 865 A1 (EVONIK OPERATIONS GMBH [DE]) 16. Dezember 2020 (2020-12-16) * Ansprüche * * Absätze [0023], [0024] * ----- | 1-15 | INV. C07C51/09 C07C67/38 C07C67/56 C07C53/126 |
| A | WO 2017/212246 A1 (IMPERIAL INNOVATIONS LTD [GB]) 14. Dezember 2017 (2017-12-14) * Absätze [0056], [0058] * ----- | 1-15 | C07C69/24 B01D61/00 |
| A | WO 2015/110843 A1 (IMP INNOVATIONS LTD [GB]) 30. Juli 2015 (2015-07-30) * Ansprüche * ----- | 1-15 | |

**RECHERCHIERTE SACHGEBIETE (IPC)**

C07C
B01D

Der vorliegende Recherchenbericht wurde für alle Patentansprüche erstellt

| Recherchenort | Abschlußdatum der Recherche | Prüfer |
|---|---|---|
| Den Haag | 25. Mai 2022 | Fitz, Wolfgang |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**     EP 21 21 5858

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-05-2022

| Im Recherchenbericht angeführtes Patentdokument | Datum der Veröffentlichung | Mitglied(er) der Patentfamilie | Datum der Veröffentlichung |
|---|---|---|---|
| EP 3750865 A1 | 16-12-2020 | CN 112321431 A | 05-02-2021 |
| | | EP 3750865 A1 | 16-12-2020 |
| | | JP 2020200327 A | 17-12-2020 |
| | | KR 20200142466 A | 22-12-2020 |
| | | SG 10202005402U A | 28-01-2021 |
| | | TW 202100503 A | 01-01-2021 |
| | | US 2020392062 A1 | 17-12-2020 |
| | | ZA 202003436 B | 28-07-2021 |
| WO 2017212246 A1 | 14-12-2017 | EP 3463630 A1 | 10-04-2019 |
| | | US 2019176092 A1 | 13-06-2019 |
| | | WO 2017212246 A1 | 14-12-2017 |
| WO 2015110843 A1 | 30-07-2015 | EP 3099400 A1 | 07-12-2016 |
| | | US 2017007963 A1 | 12-01-2017 |
| | | WO 2015110843 A1 | 30-07-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10010771 C1
- EP 1854778 A1
- EP 3121184 A2

**In der Beschreibung aufgeführte Nicht-Patentliteratur**

- **JR GAFFEN et al.** *Chem,* vol. 5 (6), 1567-1583